# FASCICULE DE BREVET EUROPEEN

(11) **EP 3 389 762 B1**
(45) Date de publication et mention de la délivrance du brevet: **04.12.2019**
(21) Numéro de dépôt: 16819447.0
(22) Date de dépôt: 09.12.2016
(51) Int. Cl.: A61N 1/05, A61B 5/00, A61B 5/04, A61N 1/36

(54) **SONDE IMPLANTABLE DE STIMULATION ÉLECTRIQUE ET/OU DE RECUEIL DE POTENTIELS ÉLECTRIQUES SUR UN ORGANE**
IMPLANTIERBARE SONDE ZUR ELEKTRISCHEN STIMULATION UND/ODER ZUM NACHWEIS VON ELEKTRISCHEN POTENTIALEN AUF EINEM ORGAN
IMPLANTABLE PROBE FOR ELECTRICAL STIMULATION AND/OR FOR DETECTION OF ELECTRICAL POTENTIALS ON AN ORGAN

(30) Priorité: 14.12.2015 FR 1562267
(43) Date de publication de la demande: 24.10.2018
(73) Titulaire: Sorin CRM SAS, 92140 Clamart (FR)
(72) Inventeur: RAULT, Maxime, 75015 Paris (FR); LE GOUSSE, Patrick, 91170 Viry Chatillon (FR)
(74) Mandataire: Grünecker Patent- und Rechtsanwälte PartG mbB
(86) Numéro de dépôt international: PCT/EP2016/080390
(87) Numéro de publication internationale: WO 2017/102578

(56) Documents cités:
- US-A1- 2002 103 511
- US-A1- 2010 262 214
- US-A1- 2013 226 272
- US-A1- 2014 228 922
- US-A1- 2014 288 577
- US-A1- 2015 066 122
- US-B1- 7 640 065

## Description

L'invention concerne les "dispositifs médicaux implantables actifs" tels que définis par la Directive 90/385/CEE du 20 juin 1990 du Conseil des communautés européennes, et plus précisément les dispositifs comprenant une ou plusieurs électrodes implantables appliquées contre un organe pour permettre la stimulation électrique de celui-ci et/ou le recueil de potentiels électriques sur cet organe.

L'invention sera plus particulièrement décrite dans le contexte de la stimulation des nerfs, spécialement la stimulation du nerf vague dans le cas de thérapies dites "VNS" (*Vagus Nerve Stimulation*).

Toutefois, cette application n'est donnée qu'à titre d'exemple et ne présente aucun caractère limitatif, l'invention pouvant être utilisée pour la stimulation/détection de tout autre organe, dans des domaines tels que neurostimulation, neurochirurgie, cardiologie, stimulation électrique de tissus, etc., chaque fois qu'il est nécessaire de disposer d'électrodes implantées comprenant une ou plusieurs interfaces avec des tissus biologiques d'un organe-cible.

Dans le cas de la stimulation des nerfs, on utilise généralement une électrode essentiellement plane que l'on vient enrouler directement autour de la paroi externe du nerf à stimuler. L'électrode est munie d'interfaces conductrices reliées à des conducteurs de liaison s'étendant ensuite le long d'un corps de sonde jusqu'à une extrémité opposée, proximale, couplée à un générateur délivrant des impulsions électriques contrôlées.

Dans d'autres applications, une électrode tubulaire est introduite à l'intérieur d'un vaisseau de manière à plaquer au plus près de la paroi interne du vaisseau les interfaces conductrices, pour mettre en contact électrique les électrodes du dispositif avec des fibres nerveuses environnantes s'étendant à proximité du vaisseau.

Dans d'autres applications encore, l'organe-cible est constitué de fibres musculaires, de tissu cérébral, de tissu cardiaque (par exemple dans une application à une électrode de défibrillation, etc.).

L'invention concerne plus particulièrement la partie de la sonde implantée contre l'organe-cible, ci-après dénommée "terminaison" ou "terminaison distale", qui permet de maintenir les électrodes au contact de l'organe-cible ou au voisinage étroit de celui-ci.

Dans l'exemple particulier de la stimulation du nerf vague, compte tenu de la configuration allongée et approximativement cylindrique du nerf, la terminaison se présente sous la forme d'un manchon de forme tubulaire, enroulé autour du nerf. Le manchon est généralement réalisé en un élastomère tel que le silicone, du fait de l'excellente biocompatibilité de ce matériau, et il porte sur sa face intérieure, appliquée contre le nerf, les électrodes de stimulation/détection.

Ce manchon est par exemple réalisé de la manière décrite par le US 4 602 624 A, à partir de deux feuilles d'élastomère laminées ensemble, l'une d'elles ayant été étirée au préalable dans une direction privilégiée de précontrainte. On réalise ainsi une terminaison auto-enroulante et auto-adaptable au diamètre du nerf : pour sa mise en place, il suffit au chirurgien de dérouler le manchon, de le passer sous le nerf puis de le relâcher pour qu'il vienne de lui-même s'enrouler autour du nerf. Si l'on choisit un diamètre intérieur au repos légèrement inférieur au diamètre du nerf, ce manchon, avec ses électrodes, restera toujours étroitement plaqué contre le nerf même si le diamètre de celui-ci varie (en effet, le nerf vague présente un diamètre moyen compris, selon les patients, entre 1,9 mm et 2,5 mm).

En ce qui concerne plus précisément les électrodes et la manière dont elles sont réalisées, une première technique, la plus répandue, consiste à rapporter des contacts et à les sertir sur le conducteur de liaison s'étendant dans le corps de sonde et assurant le couplage électrique avec le générateur d'impulsions situé à l'extrémité opposée, proximale, du corps de sonde. La résistance mécanique de l'assemblage est élevée, mais cette technologie ne permet de disposer que d'un nombre limité de contacts (habituellement 4 à 6 contacts) à la périphérie du nerf vague, du fait de la taille minimum réalisable en pratique, de l'ordre du millimètre. De plus, dans le cas de la stimulation du nerf vague, plus les contacts sont larges, moins il sera possible pour le manchon de s'enrouler au plus près du nerf.

Une autre technique consiste à réaliser les contacts à partir d'une feuille métallique maintenue à la surface du manchon silicone auto-enroulant, formant ainsi un contact annulaire avec le nerf après mise en place du manchon. L'une des limites de cette technologie tien au fait que l'électrode est annulaire et vient donc stimuler la totalité de la périphérie du nerf, sans possibilité de créer un réseau d'électrodes distinctes pour stimuler des sites ponctuels de la périphérie du nerf. Une autre difficulté réside dans la nécessité de s'assurer que la feuille métallique reste en place au contact du nerf même en cas de déformation de la terminaison.

Une troisième technique, récemment développée, consiste à déposer les contacts par dépôt chimique ou physique en phase vapeur (PVD/CVD) à la surface d'un substrat organique biocompatible, ce substrat étant ensuite fixé au manchon en silicone. L'une des difficultés réside ici dans la prise de connexion, après leur dépôt, de ces contacts avec les conducteurs de liaison, qui est une opération très délicate. De plus, la résistance mécanique de ce type d'électrode est encore très limitée, l'interface entre le substrat et le contact étant une zone sensible. Et comme il est nécessaire de permettre un enroulement de l'électrode, cet enroulement induit des cisaillements aux interfaces, qui peuvent dégrader rapidement la bonne adhésion entre les contacts et le substrat.

Les documents US 2010/0262214, US 2014/0288577, US 2002/0103511 et US 7640065 montrent différentes variantes de sondes de stimulation implantables.

En tout état de cause, aucune des techniques proposées jusqu'à présent ne procure une souplesse mécanique élevée, permettant une bonne application de la terminaison de sonde contre l'organe-cible.

Or il est éminemment souhaitable que cette terminaison de sonde (typiquement, le manchon auto-enroulable autour du nerf et les électrodes portées par ce manchon) épouse parfaitement la surface de l'organe-cible En effet, ce contact étroit est nécessaire pour abaisser le seuil électrique de stimulation et éviter ainsi l'application de courants trop élevés qui pourraient induire des effets secondaires indésirables et en tout état de cause abrégeraient la durée de vie du générateur implanté.

Une autre condition importante à respecter est que, du point de vue mécanique, la terminaison de sonde n'applique à l'organe-cible qu'un minimum de contraintes, tant au moment de l'implantation qu'après, et ceci malgré les déplacements éventuels de l'organe, son gonflement éventuel, etc. Des contraintes mécaniques exercées globalement (manchon trop serré) ou localement (au niveau d'une excroissance de la terminaison de sonde en contact avec le nerf) auraient en effet tendance à provoquer une inflammation de l'organe-cible, bien entendu dommageable.

Ces conditions sont difficilement remplies avec les terminaisons de sonde existantes, notamment :
- du fait de la raideur inévitable du manchon auto-enroulable (ou interface similaire), même si celui-ci réalisé en un matériau très souple : en effet, du fait de son caractère élastique ce manchon exerce nécessairement un effort mécanique de striction sur le nerf, effort qui de plus n'est pas uniforme et peut non générer localement des contraintes relativement importantes ;
- du fait que les électrodes conventionnelles impliquent toutes la déformation de pièces métalliques ajoutant leur rigidité propre à celle du manchon ; et
- du fait que le manchon auto-enroulable définit une surface interne de contact qui est par nature une surface développable, alors que la surface externe de l'organe-cible est une surface non développable. Même si le nerf présente une forme approximativement cylindrique, il ne s'agit là que d'une approximation et concrètement il ne pourra entrer en contact avec le manchon que de façon imparfaite, avec des régions de contact créant des contraintes mécaniques sur le nerf et des régions d'absence de contact inefficaces sur le plan de la stimulation électrique.

Un autre inconvénient de ces techniques connues est la difficulté, liée aux contraintes technologiques de réalisation, de réaliser des configurations d'électrodes complexes, par exemple des réseaux d'électrodes ponctuelles permettant de stimuler l'organe-cible en une multiplicité de sites, éventuellement sélectionnables en fonction des résultats obtenus après des tests cliniques.

L'idée de base de la présente invention consiste, au lieu d'utiliser des contacts rapportés ou déposés, à réaliser l'interface conductrice destinée à venir en contact avec l'organe-cible sous forme d'un ou plusieurs fils conducteurs longilignes assemblés par tissage, brodage, tressage ou tricotage à un substrat utilisé tel quel comme terminaison, c'est-à-dire non rapporté sur un support additionnel (par exemple sur un manchon en silicone auto-enroulable).

L'interface conductrice pourra alors s'appliquer au plus juste contre l'organe-cible, par exemple pour s'enrouler autour d'un nerf, avec une relative déformabilité permettant d'absorber les irrégularités de l'organe ou les modifications dimensionnelles (gonflements, etc.) de celui-ci.

De plus, cette configuration offre une très grande diversité dans les géométries de contacts qu'il est possible de réaliser, en tissant le ou les fils conducteurs d'une façon bien définie pour maximiser les effets recherchés et/ou définir des sites de stimulation précis sur la terminaison.

Plus précisément, l'invention propose à cet effet une sonde implantable comprenant, de manière en elle-même connue : une terminaison distale apte à venir en contact avec les tissus de l'organe, cette terminaison comprenant un substrat isolant et au moins une interface conductrice portée par le substrat ; et un corps de sonde comportant au moins un conducteur de liaison relié à une interface conductrice respective.

De façon caractéristique de l'invention, le substrat est un substrat non élastiquement déformable. De plus, l'interface conductrice est dépourvue de support additionnel élastiquement déformable incorporant le substrat ou adjoint au substrat, elle comprend au moins un fil conducteur déformable, au moins partiellement dénudé, et comprend également une configuration tissée, brodée, tressée ou tricotée d'ancrage du fil déformable au substrat. Selon diverses caractéristiques subsidiaires avantageuses :
- le substrat est une feuille de matériau homogène, ou bien une trame tissée ou tricotée, l'interface pouvant alors être incorporée à l'armure de la trame du tissu ou du tricot ;
- le fil conducteur de l'interface conductrice est distinct de son conducteur de liaison respectif, ce fil conducteur étant électriquement couplé au conducteur de liaison, et l'extrémité distale du conducteur de liaison étant mécaniquement solidarisée au substrat.

Selon un exemple ne faisant pas partie de la présente invention mais utile pour sa compréhension, le fil conducteur de l'interface conductrice est formé par un prolongement du conducteur de liaison.

On va maintenant décrire un exemple de mise en œuvre de la présente invention, en référence aux dessins annexés où les mêmes références désignent d'une figure à l'autre des éléments identiques ou fonctionnellement semblables.
La Figure 1 illustre l'ensemble des éléments d'un dispositif de stimulation VNS.
La Figure 2 illustre plus précisément un exemple de terminaison enroulable autour d'un nerf du dispositif de la Figure 1.
La Figure 3 illustre, sous forme déroulée avant mise en place, un manchon auto-enroulable selon l'état de la technique, constituant la terminaison de la Figure 2.
La Figure 4 illustre un exemple d'une terminaison utile pour la compréhension de l'invention.
La Figure 5 illustre la terminaison de la Figure 4 appliquée contre l'organe-cible.
La Figure 6 illustre un premier mode de réalisation de fixation mécanique du conducteur de liaison au substrat mettant en œuvre les enseignements de l'invention.
La Figure 7 illustre un deuxième mode de réalisation de fixation mécanique du conducteur de liaison au substrat mettant en œuvre les enseignements de l'invention.
La Figure 8 illustre un troisième mode de réalisation de fixation mécanique du conducteur de liaison au substrat mettant en œuvre les enseignements de l'invention.
La Figure 9 illustre un quatrième mode de réalisation de fixation mécanique du conducteur de liaison au substrat mettant en œuvre les enseignements de l'invention.
La Figure 10 illustre un cinquième mode de réalisation de fixation mécanique du conducteur de liaison au substrat mettant en œuvre les enseignements de l'invention.
La Figure 11 illustre un exemple de réalisation dans lequel le conducteur de liaison présente une forme hélicoïdale.
La Figure 12 est une vue de détail, fortement agrandie, de l'extrémité du conducteur de liaison de la Figure 11.
La Figure 13 illustre un autre exemple de fixation d'un conducteur de liaison hélicoïdal au substrat.
La Figure 14 illustre le substrat avant et après son application sur une surface complexe.

On va maintenant décrire divers exemples de réalisation du dispositif de l'invention.

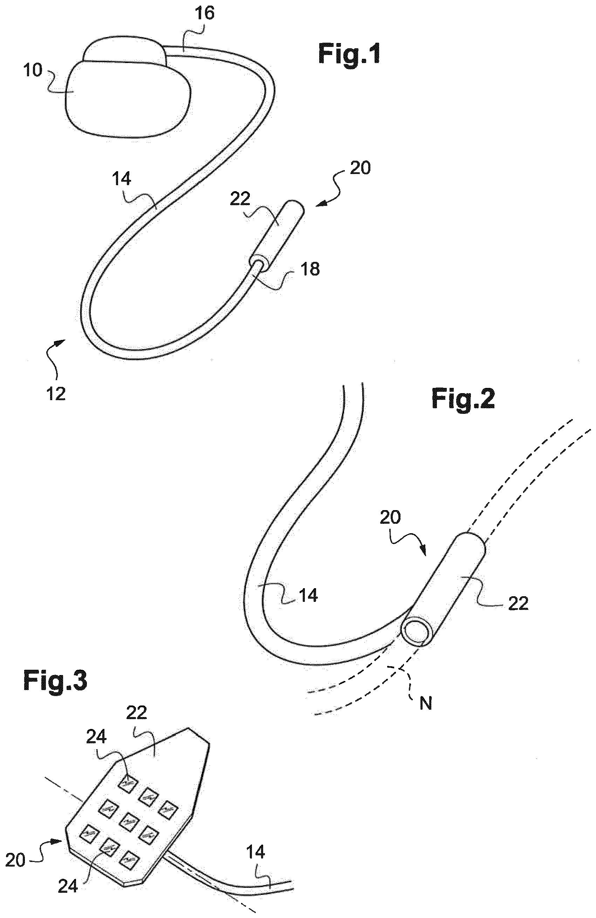

La Figure 1 illustre l'ensemble des éléments d'un dispositif de stimulation VNS.

Cet ensemble comprend un générateur d'impulsions électriques 10 relié à une (ou plusieurs) sonde(s) 12 pour la délivrance des impulsions de stimulation produites par le générateur et/ou le recueil de potentiels électriques qui seront analysés par le générateur.

La sonde 12 comprend un corps de sonde tubulaire allongé 14, flexible, relié à sa partie proximale 16 au générateur 10, généralement par l'intermédiaire d'un connecteur enfichable, et comportant à sa partie distale 18 un élément, ci-après dénommé "terminaison" 20 apte à venir en contact avec les tissus d'un organe à stimuler.

Le corps de sonde 14 inclut un certain nombre de conducteurs électriques indépendants, ci-après "conducteurs de liaison" permettant de relier diverses électrodes de la terminaison 20 à des bornes homologues du générateur 10. Ces conducteurs de liaison sont généralement enroulés à l'intérieur du corps de sonde avec une configuration hélicoïdale, de manière à préserver la flexibilité de la sonde en conservant des propriétés de résistance mécanique suffisantes pour éviter toute rupture d'un conducteur de liaison.

La terminaison 20 peut prendre plusieurs formes, et l'on décrira ci-après plus précisément une configuration où la terminaison est enroulable autour d'un nerf, typiquement le nerf vague.

La Figure 2 illustre un exemple de terminaison conventionnelle, réalisée au moyen d'un manchon 22 auto-enroulable autour du nerf N de manière à épouser au mieux la forme de celui-ci.

La Figure 3 illustre, sous forme déroulée avant mise en place, le manchon de la terminaison de la Figure 2.

Ce manchon 22 est typiquement réalisé à partir d'une feuille en matériau élastiquement déformable, par exemple un silicone, portant une pluralité de plages électriquement conductrices (ci-après "interfaces conductrices") destinées à venir s'appliquer contre un site de stimulation précis de l'organe à stimuler. Chacune des interfaces conductrices 24 est reliée à un conducteur respectif courant à l'intérieur du corps de sonde 14 pour être relié à une borne correspondante du générateur, côté proximal. La feuille déformable destinée à former le manchon 22 est avantageusement précontrainte de manière à permettre son auto-enroulement depuis une position initiale où la feuille est maintenue sous contrainte à l'état déployé (configuration de la Figure 3) jusqu'à une position finale où la feuille est librement enroulée en spirale en formant un manchon autour de l'organe (configuration de la Figure 2), la face portant des interfaces conductrices 24 venant alors en appui contre la surface extérieure du nerf N. Une telle configuration de manchon auto-enroulable est par exemple divulguée dans le US 4 602 624 A précité.

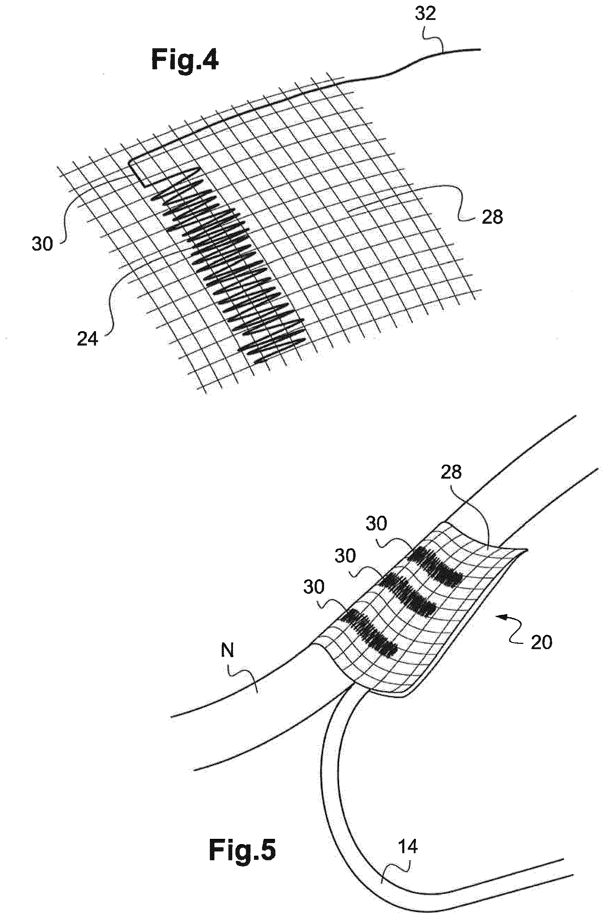

La Figure 4 illustre un exemple de terminaison ne faisant pas partie de la présente invention mais utile pour sa compréhension.

De façon caractéristique de l'invention, chacune des interfaces conductrices 24 est réalisée à partir d'un fil conducteur déformable 30, au moins partiellement dénudé et qui, dans l'exemple illustré, est ancré à un substrat 28 par brodage ou par une autre technique analogue (tissage, tressage, tricotage, etc.). Ce fil conducteur 30 est relié à un conducteur de liaison respectif 32. L'ensemble des conducteurs de liaison 32 de la terminaison court ensuite à l'intérieur du corps de sonde jusqu'au générateur distant situé côté proximal. Dans l'exemple de terminaison illustré Figure 4, le fil conducteur 30 est constitué par un prolongement direct du conducteur de liaison, et il est brodé sur le substrat 28. Le fil subsistant après brodage est conservé et constitue le conducteur de liaison 32, et il n'y a ainsi aucune solution de continuité entre ces deux éléments.

La Figure 5 illustre la terminaison de la Figure 4, appliquée contre l'organe-cible. Comme on peut le constater, le substrat tissé isolant 28, qui est très souple et déformable, peut être utilisé à la manière d'une gaze appliquée et enroulée autour du nerf N. Les régions où ont été brodés les fils conducteurs 30 forment des électrodes périphériques de stimulation du nerf, et la partie débordante du substrat 28 (partie qui s'étend approximativement dan un plan axial) se raccorde le corps de sonde 14 qui n'est donc pas directement en contact avec le nerf N, ce qui réduit très fortement la transmission au nerf N de contraintes susceptibles d'être reçues par le corps de sonde 14.

De façon générale, le substrat 28 peut se présenter sous différentes formes, en fonction des spécificités de l'organe à stimuler.

Selon le cas, le substrat 28 peut être réalisé à partir de : un matériau homogène isotrope ; un matériau homogène anisotrope ; un matériau à fibres courtes globalement isotrope ; un matériau à fibres courtes globalement anisotrope ; un matériau tissé unidirectionnel ; un matériau tissé bidirectionnel ; un matériau tissé multidirectionnel ; ou toute combinaison des précédents.

En ce qui concerne le matériau proprement dit du substrat, celui-ci peut être : un matériau actif tel que le nitinol ; un matériau en polymère biocompatible tel que le dacron, le silicone, le parylène, le polyéthylène, le polyuréthane ou le polyamide ; un matériau conducteur tel que le MP35N, le platine, un alliage de platine-iridium, un acier inox tel que 304 ou 316 ; un matériau résorbable ; un matériau soluble tel que le glucose ; ou toute combinaison des précédents.

Le substrat peut être déformable de façon réversible ou non, avec ou sans apport d'énergie d'origine mécanique, thermique ou chimique ou autre source d'énergie extérieure ou intérieure au substrat.

Dans le cas d'un substrat tissé, les fils de trame et de chaine qui le composent peuvent ou non bouger les uns par rapport aux autres. Toujours dans le cas d'un substrat tissé, les fils peuvent être tissés à angle droit ou selon un autre angle (60°, 45°, 30°, 15° ou tout autre angle). D'autre part, l'armure du tissage peut être de tout type connu tel que sergé, satin, taffetas ou tout autre tissage.

Toujours dans le cas d'un substrat tissé, il est possible d'employer un tissu de fils conducteurs ou non, isolés ou non. Si l'on utilise des fils conducteurs incorporés au substrat, ceux-ci peuvent être dénudés sur toute leur longueur, ou bien uniquement à certains endroits. Dans ce dernier cas, le dénudage peut être effectué mécaniquement, par ablation laser, par ablation chimique, par abrasion ou toute autre méthode permettant d'exposer au milieu extérieur au moins une partie du matériau conducteur du fil. On notera qu'il n'est pas nécessaire que l'ensemble des fils de trame ou de chaine soient conducteurs, mais que cette propriété peut être réservée à certains seulement des fils de trame et/ou de chaine, les autres étant des fils non conducteurs conventionnels, formant le substrat.

Enfin, toujours dans le cas d'un substrat tissé, les fils conducteurs formant l'interface conductrice peuvent être incorporés à la trame formant le substrat au moment du tissage ou du tricotage de celle-ci, ou bien ajoutés au substrat dans une étape ultérieure, par exemple par brodage, le fil conducteur traversant alors le substrat et étant ainsi mécaniquement ancré à ce dernier. L'interface conductrice destinée à venir en contact avec les tissus de l'organe à stimuler est ainsi cousue, tissée ou brodée sur le substrat. Le positionnement et l'étendue de l'interface conductrice pourront alors être judicieusement choisis et orientés de manière que le substrat reste flexible et/ou extensible, la terminaison restant ainsi globalement flexible et/ou extensible, cette propriété étant nécessaire pour épouser au mieux l'organe à stimuler et s'adapter aux déformations éventuelles de celui-ci.

L'interface conductrice pourra comporter un substrat avec une orientation de tissage, tressage, broderie ou toute autre méthode similaire correctement choisie, équipée d'électrodes tissées, brodées, cousues, ou assemblée par toute autre méthode similaire à même de pouvoir épouser des formes complexes non développables.

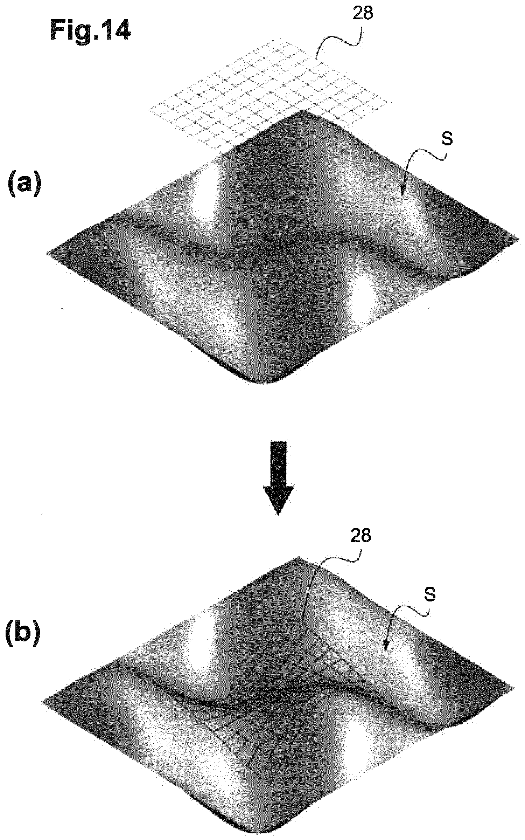

Sur la Figure 14, on a illustré en (a) le substrat 28 avant son application sur la surface réceptrice S, et en (b) après son application : on peut voir que le substrat 28 épouse parfaitement la forme complexe S en permettant à ses constituants (fils de trame et de chaine, etc.) de se repositionner les uns par rapport aux autres. Il n'y a ainsi pas d'effort permanent induit sur les tissus biologiques, tout en gardant un contact optimal.

Le fil conducteur constituant l'interface conductrice peut être un fil isolé ou non, monobrin ou multibrin. S'il est isolé, l'âme conductrice sera exposée au milieu extérieur par ablation laser, ablation chimique, découpe manuelle ou tout autre moyen permettant de dénuder le conducteur.

Dans l'exemple illustré sur la Figure 4, le fil conducteur constituant l'interface conductrice de la terminaison était un prolongement du fil de liaison, celui-ci étant alors mécaniquement lié au substrat par un moyen approprié (points de couture, points de colle, etc.).

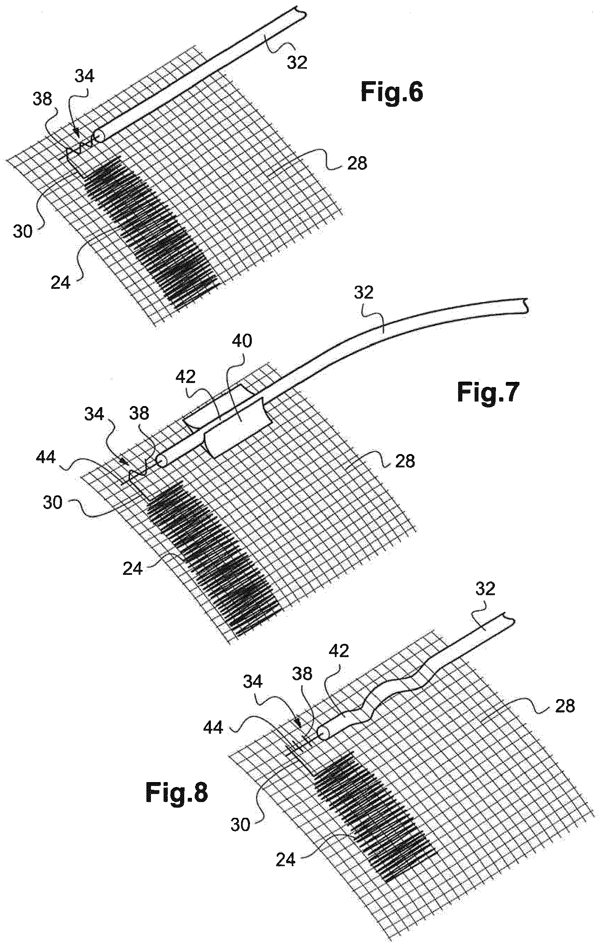

Dans des formes de réalisation de l'invention, illustrées Figures 6 à 13 et décrites ci-après, le fil conducteur est distinct du fil de liaison, et ces deux éléments sont électriquement reliés au niveau d'un point de couplage. Ceci permet notamment d'utiliser pour le fil conducteur constituant l'interface conductrice un fil très fin, le fil de liaison étant, quant à lui, plus épais.

En ce qui concerne le conducteur de liaison, si celui-ci est de nature globalement longiligne, il peut être, au moins partiellement, lié mécaniquement au substrat par couture, tissage, brodage directement ou avec un matériau tiers. Les points ainsi formés peuvent être continus, espacés régulièrement ou espacés irrégulièrement.

Le conducteur de liaison, ou une partie de celui-ci, peut être lié mécaniquement au substrat par une colle, une soudure, une brasure, une fusion locale ou tout autre procédé mettant en œuvre les propriétés cohésives ou adhésives d'une matière tierce ou autogène.

Dans le cas d'un conducteur de liaison de nature globalement longiligne et avec un substrat plan, il est possible de solidariser le conducteur de liaison au substrat en croisant une ou plusieurs fois le plan formé par le substrat, c'est-à-dire à la manière d'un point de couture passant alternativement par-dessus et par-dessous le substrat.

Toujours dans le cas d'un conducteur de nature globalement longiligne et avec un substrat plan, une méthode pour le lier mécaniquement, en tout ou partie, avec le substrat consiste à effectuer un ou plusieurs virages globalement dans le plan du substrat, avec des rayons de courbure plus ou moins importants.

Si le conducteur de liaison est de nature globalement longiligne, ce conducteur ou une partie de celui-ci peut être lié mécaniquement au fil conducteur constituant l'interface conductrice par soudure, brasure, collage, coincement, brodage, tissage, tressage, couture, sertissage, liage ou par toute autre méthode d'assemblage.

Si le conducteur de liaison est de nature hélicoïdale et si le substrat est plan, une méthode pour le lier mécaniquement, ou bien en lier une partie, peut amener une ou plusieurs spires d'un ou plusieurs brins de l'hélice à être déformées de manière à rendre ces brins parallèles au plan du substrat, auquel ils pourront alors être liés mécaniquement sur tout ou partie de leur longueur par soudure, brasure, collage, coincement, brodage, tissage, tressage, couture, sertissage, liage ou par tout autre procédé.

Les conducteurs, qu'ils soient utilisés pour réaliser les conducteurs de liaison ou bien les conducteurs d'électrodes peuvent être de plusieurs natures. Ils peuvent soient être naturellement biocompatible soit enduits de matériaux biocompatibles tels que le 35NLT, le MP35N, l'acier inoxydable 304, l'acier inoxydable 316L, le platine, l'alliage de platine-iridium, ou tout autre métal biocompatible, ou tout autre matériau conducteur ou semi-conducteur biocompatible. Ces conducteurs peuvent être isolés ou non. Si tel est le cas, le matériau d'isolement peut être par exemple de l'éthylène tétrafluoroéthylène (ETFE).

La Figure 6 illustre un premier mode de réalisation de fixation mécanique du conducteur de liaison au substrat, au niveau d'un point de couplage 34.

Dans cet exemple, le conducteur de liaison 32, qui est électriquement relié au fil conducteur 30 brodé sur le substrat 28, est ancré mécaniquement à ce dernier par des points de couture 38.

La Figure 7 illustre un deuxième mode de réalisation de fixation mécanique du conducteur de liaison au substrat.

Dans cet exemple, le conducteur de liaison 32 est ancré au substrat 28 par l'intermédiaire d'un point de colle 40 venant entourer l'isolant 42 (ou la partie dénudée) du conducteur de liaison 32 dans une région voisine du point de couplage avec le fil conducteur 30, l'âme conductrice 44 du conducteur de liaison étant, comme dans l'exemple de la Figure 6, ancrée mécaniquement au substrat 28 par des points de couture 38, de manière à assurer une reprise des contraintes éventuelles subies dans la région du couplage avec le fil conducteur 30.

La Figure 8 illustre un troisième mode de réalisation de fixation mécanique du conducteur de liaison au substrat.

Dans cet exemple, le conducteur de liaison 32 est solidarisé au substrat 28 par tissage avec celui-ci, en passant alternativement au-dessous et au-dessus du plan de ce substrat. L'ancrage du conducteur de liaison 32 au substrat 28 est ainsi assuré de façon purement mécanique, donc sans recours à un matériau tiers comme dans le cas du collage 40 de la Figure 7. Pour le reste, la réalisation est identique à celle de la Figure 7, les points de couture 38 pour l'ancrage de l'âme conductrice 44 au substrat 28 étant situés au voisinage de la liaison de celle-ci avec le fil conducteur 30.

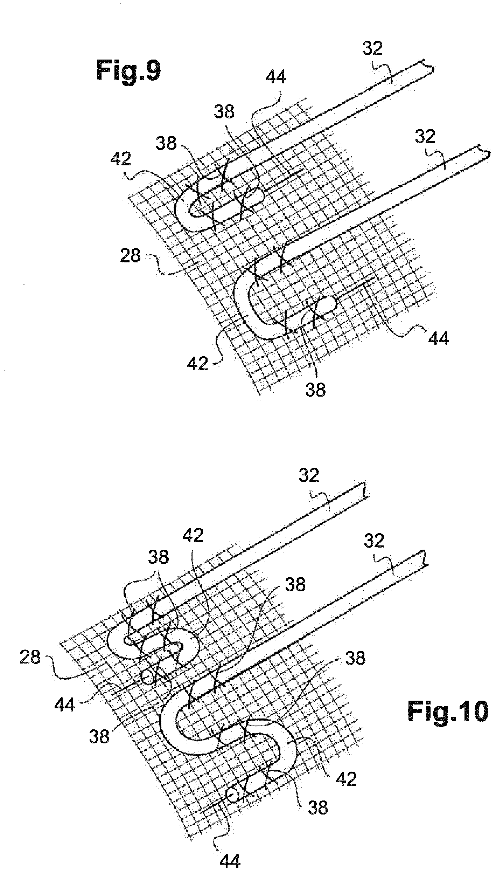

La Figure 9 illustre un quatrième mode de réalisation de fixation mécanique du conducteur de liaison au substrat.

Dans cet exemple, le conducteur de liaison 32 se termine en forme de U dans sa partie d'extrémité distale, juste avant la région d'émergence de l'âme conductrice 44. La gaine isolante 42 (ou la partie dénudée) du conducteur de liaison 32 est alors solidarisée au substrat 28 par des points de couture 38 réalisés sur chacune des branches du U.

La Figure 10 illustre un cinquième mode de réalisation de fixation mécanique du conducteur de liaison au substrat.

Dans cet exemple, la partie terminale du conducteur de liaison 32 est configurée en forme de S dans sa partie d'extrémité distale, juste avant la région d'émergence de l'âme conductrice 44. La gaine isolante 42 (ou la partie dénudée) du conducteur de liaison 32 est alors solidarisée au substrat 28 par des points de couture 38 réalisés sur chacune des branches du S.

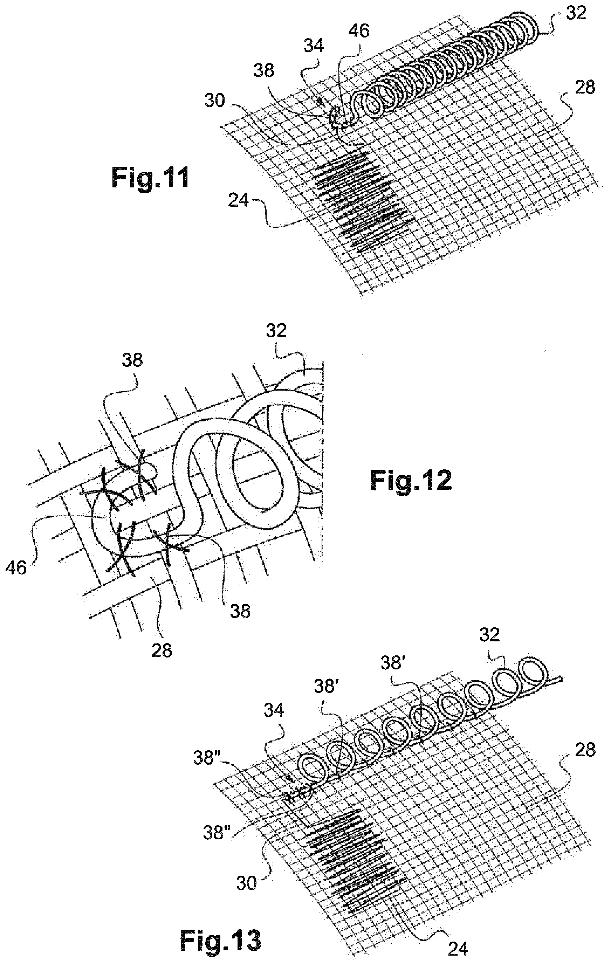

La Figure 11 illustre un exemple dans lequel le conducteur de liaison 32 présente une forme hélicoïdale, correspondant typiquement au cas d'un conducteur de liaison hélicoïdal émergeant de l'intérieur du corps de sonde et qui n'est pas redressé dans sa partie terminale. L'extrémité libre du conducteur de liaison est alors configurée par exemple en forme de boucle ou crochet 46, qui est solidarisé au substrat 28 par des points de couture 38, dans la région située au voisinage du couplage avec le fil conducteur 30 qui formera l'interface conductrice de la terminaison de sonde.

La Figure 12 est une vue de détail, fortement agrandie, de l'extrémité du conducteur de liaison de la Figure 11, montrant le crochet 46 et les points de couture 38 pour sa fixation au substrat 28.

La Figure 14 illustre encore un autre mode de fixation d'un conducteur de liaison hélicoïdal au substrat.

Dans cet exemple, les dernières spires du conducteur de liaison 32 sont ancrées au substrat par des points de couture 38' dans la région où ces spires viennent en contact avec celui-ci, la partie terminale du conducteur de liaison 32 étant quant à elle ancrée à ce même substrat par d'autres points de couture 38" au voisinage de la zone de couplage avec le fil conducteur 30 destiné à former l'interface conductrice de la terminaison.

## Revendications

1. Une sonde implantable (12) pour la stimulation électrique d'un organe (N) et/ou le recueil de potentiels électriques sur cet organe, comprenant :
- une terminaison (20) distale apte à venir en contact avec les tissus de l'organe, cette terminaison comprenant :
• un substrat (28) isolant, et
• au moins une interface conductrice (24) portée par le substrat ; et
- un corps de sonde (14), comportant au moins un conducteur de liaison (32) relié à une interface conductrice respective,
sonde **caractérisée en ce que** :
- le substrat (28) est un substrat non élastiquement déformable ;
- l'interface conductrice (24) est dépourvue de support additionnel élastiquement déformable incorporant le substrat ou adjoint au substrat ;
- l'interface conductrice (24) comprend au moins un fil conducteur (30) déformable, au moins partiellement dénudé ; et
- l'interface conductrice (24) comprend une configuration tissée, brodée, tressée ou tricotée d'ancrage du fil déformable (30) au substrat (28) ;
- le fil conducteur (30) de l'interface conductrice est distinct de son conducteur de liaison (32) respectif, le fil conducteur étant électriquement couplé (34) au conducteur de liaison, et l'extrémité distale du conducteur de liaison étant mécaniquement solidarisée (38, 40) au substrat.

2. La sonde de la revendication 1, dans laquelle le substrat est une feuille de matériau homogène.

3. La sonde de la revendication 1, dans laquelle le substrat est une trame tissée ou tricotée.

4. La sonde de la revendication 3, dans laquelle l'interface est incorporée à l'armure de la trame du tissu ou du tricot.

## Patentansprüche

1. Implantierbare Sonde (12) zur elektrischen Stimulation eines Organs (N) und/oder zur Erfassung elektrischer Potentiale an dem betreffenden Organ mit:
- einem distalen Ende (20), das mit dem Gewebe des Organs in Kontakt kommen kann, wobei dieses Ende :
• ein isolierendes Substrat (28) und
• mindestens eine leitfähige Kontaktfläche (24), die auf dem Substrat aufgebracht ist, umfasst; und
- einem Sondenkörper (14), der mindestens einen Verbindungsleiter (32) umfasst, welcher mit einer entsprechenden leitfähigen Kontaktfläche verbunden ist,
Sonde **dadurch gekennzeichnet, dass**:
- das Substrat (28) ein nicht elastisch verformbares Substrat ist;
- die leitfähige Kontaktfläche (24) frei von einem zusätzlichen, elastisch verformbaren Träger ist, der das Substrat beinhaltet oder an dem Substrat haftet;
- die leitfähige Kontaktfläche (24) mindestens einen verformbaren, zumindest teilweise abisolierten Leitdraht (30) umfasst; und
- die leitfähige Kontaktfläche (24) eine gewebte, gestickte, geflochtene oder gewirkte Struktur des verformbaren Drahtes (30) auf dem Substrat (28) umfasst;
- der Leitdraht (30) der leitfähigen Kontaktfläche von seinem jeweiligen Verbindungsleiter (32) abgegrenzt ist, wobei der Leitdraht mit dem Verbindungsleiter elektrisch gekoppelt (34) ist, und das distale Ende des Verbindungsleiters am Substrat mechanisch befestigt (38, 40) ist.

2. Sonde nach Anspruch 1, bei der das Substrat eine Folie aus homogenem Material ist.

3. Sonde nach Anspruch 1, bei der das Substrat ein gewebtes oder gewirktes Geflecht ist.

4. Sonde nach Anspruch 3, bei der die Kontaktfläche in das Fadengeflecht des Gewebes oder Gewirkes integriert ist.

## Claims

1. An implantable lead (12) for electrically stimulating an organ (N) and/or for collecting electrical potentials on said organ, the implantable lead comprising:
- a distal ending (20) suitable for coming into contact with the tissues of the organ, this ending comprising:
• an insulating substrate (28); and
• at least one conductive interface (24) carried by the substrate; and
- a lead body (14), including at least one link conductor (32) connected to a respective conductive interface;
said implantable lead being **characterized in that**:
- the substrate (28) is a substrate that is not elastically deformable;
- the conductive interface (24) is devoid of any additional elastically deformable support incorporating the substrate or added to the substrate;
- the conductive interface (24) comprises at least one deformable conductive wire (30) that is at least partially bared; and
- the conductive interface (24) further comprises a woven, embroidered, braided, or knitted configuration for anchoring the deformable wire (30) to the substrate (28); the conductive wire (30) of the conductive interface is distinct from its respective link conductor (32), the conductive wire being electrically coupled (34) to the link conductor, and the distal end of the link conductor being mechanically secured (38, 40) to the substrate.

2. The lead of claim 1, wherein the substrate is a homogeneous sheet of material.

3. The lead of claim 1, wherein the substrate is a woven or knitted fabric mesh.

4. The lead of claim 3, wherein the interface is incorporated into the structure of the mesh of the woven or knitted fabric.
